Europäisches Patentamt

European Patent Office

Office européen des brevets

(11) Veröffentlichungsnummer: **0 261 317 B1**

(12) **EUROPÄISCHE PATENTSCHRIFT**

(45) Veröffentlichungstag der Patentschrift: **28.08.91**

(51) Int. Cl.⁵: **F16K 15/18, F16K 15/14**

(21) Anmeldenummer: **87108969.4**

(22) Anmeldetag: **23.06.87**

(54) **Rückschlagventilvorrichtung.**

(30) Priorität: **25.09.86 US 911419**

(43) Veröffentlichungstag der Anmeldung:
**30.03.88 Patentblatt 88/13**

(45) Bekanntmachung des Hinweises auf die
Patenterteilung:
**28.08.91 Patentblatt 91/35**

(84) Benannte Vertragsstaaten:
**AT BE CH DE ES FR GB GR IT LI NL SE**

(56) Entgegenhaltungen:
**DE-C- 3 100 442**
**US-A- 3 570 484**
**US-A- 4 143 853**
**US-A- 4 387 879**
**US-A- 4 535 820**

(73) Patentinhaber: **B. Braun Melsungen AG**
**Carl-Braun Strasse**
**W-3508 Melsungen(DE)**

(72) Erfinder: **Raines, Kenneth**
**1727 Markham Drive**
**Bethlehem Pennsylvania 18017(US)**

(74) Vertreter: **Selting, Günther, Dipl.-Ing. et al**
**Deichmannhaus am Hauptbahnhof**
**W-5000 Köln 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Rückschlagventilvorrichtung der im Oberbegriff des Anspruches 1 definierten Art.

Ein gemeinsames Problem herkömmlicher Rückschlagventilvorrichtungen, die normalerweise geschlossen sind, besteht darin, daß beim Rückstrom von Flüssigkeit durch einen Schlauch von einer Injektionsspritze oder dergleichen der Ventilkörper nicht schnell genug seine Schließstellung einnimmt, so daß die Gefahr eines Flüssigkeitsrückstromes vom Auslaß zum Einlaß besteht, der ungünstigenfalls für den Patienten sogar tödlich sein kann. Ferner fehlt häufig die Möglichkeit, vor Enleitung eines Flüssigkeitsstromes mit zur Betätigung des Ventilkörpers ausreichendem Strömungsdruck, den Ventilkörper durch äußere Einwirkung in Öffnungsstellung bringen zu können.

Die Rückschlagventilvorrichtung gemäß dem Oberbegriff des Anspruches 1 (US-A-45 35 820) arbeitet mit einer Ventilscheibe aus elastischem Material, die normalerweise ihre Schließstellung innehat, bei Auftreffen eines Flüssigkeitsstromes gegen ihre eine Seitenfläche durch Randdeformation den Durchlaßkanal öffnet und infolge des kurzen Rückstellweges des Ventilscheibenrandes bei Flüssigkeitsrückstrom schnell ihre Schließstellung wieder einnimmt. Diese Rückschlagventilvorrichtung läßt sich nicht beliebig öffnen, um in die Leitung, in die die Rückschlagventilvorrichtung eingesetzt ist, beispielsweise mit einer Spritze, Medikamente einzuspritzen oder aus dieser abzuziehen.

Eine andere Rückschlagventilvorrichtung ist im Zusammenhang mit einem Ansatz für eine Venenverweilkanüle für Infusionen bekannt (US-A-3 570 484). Um nach der Gefäßpunktion den Punktionserfolg durch sichtbaren Blutrückfluß erkennen zu können, wird hierbei ein in den Durchlaßkanal eines Gehäuses eingesetzter Gummistöpsel mit Hilfe eines gegen einen durchbohrten Kolben drückenden Spritzenkegels gegen Federkraft vom Ventilsitz abgehoben. Während der Infusion hält der Flüssigkeitsstrom die Rückschlagventilvorrichtung geöffnet; hierzu ist beträchtlicher Flüssigkeitsdruck erforderlich, weil die auf den Gummistöpsel wirkende Gegenkraft der Feder überwunden werden muß, die zur Erzielung ausreichender Schließgeschwindigkeit bei Flüssigkeitsrückstrom verhältnismäßig groß sein muß. Flüssigkeitsdruckschwankungen können zum Ventilverschluß und zu Infusionsunterbrechungen mit unabsehbaren Folgen für den Patienten führen.

Ferner ist es aus der DE-C-3100442 bekannt, bei einem Anschlußstück für Kunststoffkanülen und Venenkatheter eine Ventilvorrichtung vorzusehen, die eine mit ihrem Rand in das Gehäuse fest eingespannte, zentralgeschlitzte Ventilscheibe und ein rohrförmiges Betätigungselement aufweist. Das rohrförmige Betätigungselement ist in dem Durchlaßkanal des Gehäuses axial verschiebbar und drückt mit seiner einen Stirnfläche gegen das Zentrum der Ventilscheibe, wobei es den Schlitz zu seiner Öffnung aufspreizt und die Ventilscheibe zumindest teilweise durchsetzt. Das Betätigungselement wird von einem Spritzenkegel verschoben und soll nach Zurückziehung des Spritzenkegels durch die elastische Rückstellfähigkeit des Materials der Ventilscheibe in unwirksame Position zurückgeschoben werden.

Der Erfindung liegt die Aufgabe zugrunde, eine Rückschlagventilvorrichtung zu schaffen, die sich bei geringem Flüssigkeitsdruck öffnet, bei Flüssigkeitsrückstrom schnell schließt und mittels einer Spritze zum Zuspritzen oder Abziehen von Fluiden beliebig öffenbar ist.

Diese Aufgabe wird durch die im Kennzeichen des Anspruches 1 aufgeführten Merkmale gelöst.

Die flexible, elastische Ventilscheibe, die durch zentrale Halterung daran gehindert wird, sich aus der Mittelachse der Kammer seitlich zu bewegen, kann durch Deformation ihres Randbereiches unter Flüssigkeitsdruck bei der Infusion oder vor Ankopplung der Infusionsvorrichtung unter dem Einfluß des betätigten Bügels geöffnet werden. Der einfache Aufbau der Rückschlagventilvorrichtung gewährleistet verklemmungsfreie, schnell ansprechende Funktion sowohl bei der Infusion als auch bei Öffnung durch Bügelbetätigung mit Hilfe eines gegen den Bügel drückenden Kegels einer Spritze zum Zuspritzen oder Abziehen von Fluiden. Die Rückschlagventilvorrichtung wird bei geringem Flüssigkeitsdruck sofort geöffnet und schließt sich bei Flüssigkeitsrückstrom unverzüglich.

Vorteilhafte Ausgestaltungen des Bügels sind in dem Anspruch 2 enthalten. Die Stirnfläche des Spritzenkegels wird gegen die von den Schenkeln freie Seite des Ringes gedrückt, so daß der Bügel axial verschoben wird und mit seinen Schenkelenden den Randbereich der Ventilscheibe zur Öffnung des Durchlaßkanals verformt. Nach Zuspritzen oder Abziehen von Flüssigkeit, die durch das Loch des als Ring ausgebildeten Bügelsteges ungehindert hindurchströmen kann, wird die Spritze entfernt und der Randbereich der Ventilscheibe schnellt in seine im wesentlichen ebene Grundform zurück. Die Ventilscheibe nimmt ihre Schließstellung ein, die sie erst wieder unter dem Einfluß des Infusionsflüssigkeitsstromes aufgibt.

Diese Merkmale sichern den Bügel gegen axiales Herausziehen oder Herausfallen aus dem Einlaßabschnitt des Durchlaßkanals, weil bei unbetätigtem Bügel die Rippen die Oberkante der Umfangsauskehlung untergreifen. Die Umfangsauskehlung ist gegen die Kammer offen und gestattet einen freien Austritt der Schenkel bei Bügelbetätigung, so daß sie den Randbereich der Ventilscheibe von der als Ventilsitz dienenden Unterkante der Umfangsauskehlung wegdrücken können.

Einzelheiten des Gesamtaufbaus der Rückschlagventilvorrichtung ergeben sich aus den Merkmalen des Anspruches 3. Die beiden ineinandergesteckten becherförmigen Ansätze der zwei Gehäuseteile bilden eine zylindrische Kammer, in der die Ventilscheibe untergebracht ist. Ihre Mittelzone liegt punktförmig auf der Spitze des Plattenkörpers auf und ist zwischen dieser und dem Querbalken eingespannt. Der Druck zwischen der Spitze des Plattenkörpers und dem Querbalken reicht aus, um die biegsame Ventilscheibe an Seitenbewegungen zu hindern und zentriert festzuhalten. Ihr Randbereich bleibt von der Halterung frei und kann sich in beiden Richtungen quer zur Scheibenebene bewegen. Der Durchlaßkanal ist maximal geöffnet, wenn die Ventilscheibe so gewölbt ist, daß ihre eine Seite gegen die beiden Dreieckseiten des Plattenkörpers anliegt. In diesem Zustand bildet der Plattenkörper einen Anschlag, der den Anwender am weiteren Vorschub des Spritzenkegels hindert und die Scheibenverformung auf ein zulässiges Maß beschränkt.

Als zusätzliche Maßnahme zur Verhinderung des Anhaftens des Umfangsrandes der Ventilscheibe an der Wand der Kammer sind an ihrer Innenwand längs verlaufende Rippen vorgesehen, die die Ventilscheibe als Kranz sehr eng umgeben. Diese Rippen, die einen weiten Abstand zueinander haben und verhältnismäßig schmal sind, behindern den Flüssigkeitsdurchstrom nicht, tragen aber erheblich zu einer ungehinderten, flexiblen und anhaftungsfreien Bewegung des Ventilscheibenrandes bei.

Die Merkmale des Anspruches 4 erlauben zusätzlich zu der Beobachtung des Flüssigkeitsstromes ein gezieltes Ansetzen des Spritzenkegels gegen den Ring des Bügels.

Im folgenden wird eine Ausführungsform der Erfindung unter Bezugnahme auf die Zeichnungen näher erläutert. Es zeigen:

Fig. 1 eine perspektivische Außenansicht der Rückschlagventilvorrichtung im zusammengefügten Zustand;

Fig. 2 eine explodierte perspektivische Ansicht der Einzelteile der Rückschlagventilvorrichtung und

Fig. 3 einen Querschnitt längs der Linie 3-3 in Figur 1.

Die normalerweise im Schließzustand befindliche Rückschlagventilvorrichtung weist ein aus zwei Gehäuseteilen 11 und 25 zusammengesetztes Ventilgehäuse 10 auf. Der Gehäuseteil 11 besteht aus einem rohrförmigen Stutzen 12, an dessen eines Ende ein zylindrischer becherförmiger Ansatz 16 angeformt ist, dessen Innen- und Außendurchmesser größer als die entsprechenden Durchmesser des Stutzens 12 sind. Am anderen Ende des Stutzens 12 befinden sich nach außen gerichtete radiale Verriegelungsnocken 13. Die Innenfläche und die Außenfläche der Wand 57 des Ansatzes 16 sind kreiszylindrisch, während der kreisförmige Einlaßabschnitt 17 eines Durchlaßkanals 117 in dem Stutzen 12 mit gegen den Ansatz 16 gerichteter Verjüngung konisch verläuft. Auf der Innenfläche der Wand 57 des Ansatzes 16 sind mehrere axiale Rippen angeordnet, die kürzer als die Wand 57 und mit großen gegenseitigen Abständen über ihren Umfang verteilt angeordnet sind. Die Wand 57 des Ansatzes 16 ist über einen radial einwärts gerichteten Übergangsteil 18 an den Stutzen 12 angeschlossen. Die Innenfläche des Übergangsteils 18 verläuft im wesentlichen senkrecht zur Innenfläche der Wand 57 des Ansatzes 16 und bildet eine ebene Schulter 14. In der der inneren Mündung des Einlaßabschnittes 17 zugewandten Kante der Schulter 14 ist eine Umfangsauskehlung 166 ausgebildet, deren Ober- und Unterkante radial zueinander so versetzt sind, daß die Auskehlung gegen den Ansatz 16 vorsprungslos offen ist. In der Mündung des Einlaßabschnittes 17 ist ein Querbalken 15 vorgesehen, dessen Enden an der Innenfläche des Stutzens 12 befestigt sind. Der Querbalken 15 ist an seinem dem Ansatz 16 zugewandten Rand im wesentlichen eben, während sein gegenüberliegender Rand zur Erleichterung der Flüssigkeitsumströmung abgerundet ist. Der ebene Rand des Querbalkens 15 liegt im wesentlichen in der Ebene der Oberkante der Umfangsauskehlung 166.

Der andere Gehäuseteil 25 des Ventilgehäuses 10 besteht aus einem Außenkonus 30 mit einem kreiszylindrischen Auslaßabschnitt 37 des Durchlaßkanals 117, der zu dem Einlaßabschnitt 17 koaxial ausgerichtet ist. Der Außenkonus 30 ist mit Abstand von einer konzentrischen Hülse 22 umgeben, auf deren kreiszylindrischer Innenfläche 31 ein Innengewinde 34 mit großer Steigung ausgebildet ist, das der Aufnahme von Verriegelungsnocken eines angesetzten Innenkonus einer zu einem Patienten führenden Leitung dient. Auf der Außenfläche der Hülse 22 sind abwechselnd breite Längsrippen und schmale Längsrillen zur Verbesserung der Griffigkeit beim Zusammenschrauben von Teilen angebracht. An die Hülse 22 und den Außenkonus 30 ist über einen radial nach außen gerichteten Übergangsteil einstückig ein becherförmiger Ansatz 26 angeformt, dessen Wand 28 auf der Innenfläche und auf der Außenfläche kreiszylindrisch gestaltet ist. Der Außendurchmesser des Ansatzes 16 ist geringfügig kleiner als der Innendurchmesser des Ansatzes 26, so daß beide Ansätze passend zusammengesteckt und durch Ultraschallverschweißung fest und dicht miteinander verbunden werden können. Die beiden zusammengesteckten Ansätze 16 und 26 bilden eine Kammer 52 mit kreiszylindrischer Umfangsfläche, einem ebenen Boden 24 und einer zu diesem parallelen durch die Schulter 14 geformten ringartigen Oberwand. Der Durchmesser der Kammer 52 ist größer als derjenige des Durchlaßkanals 117.

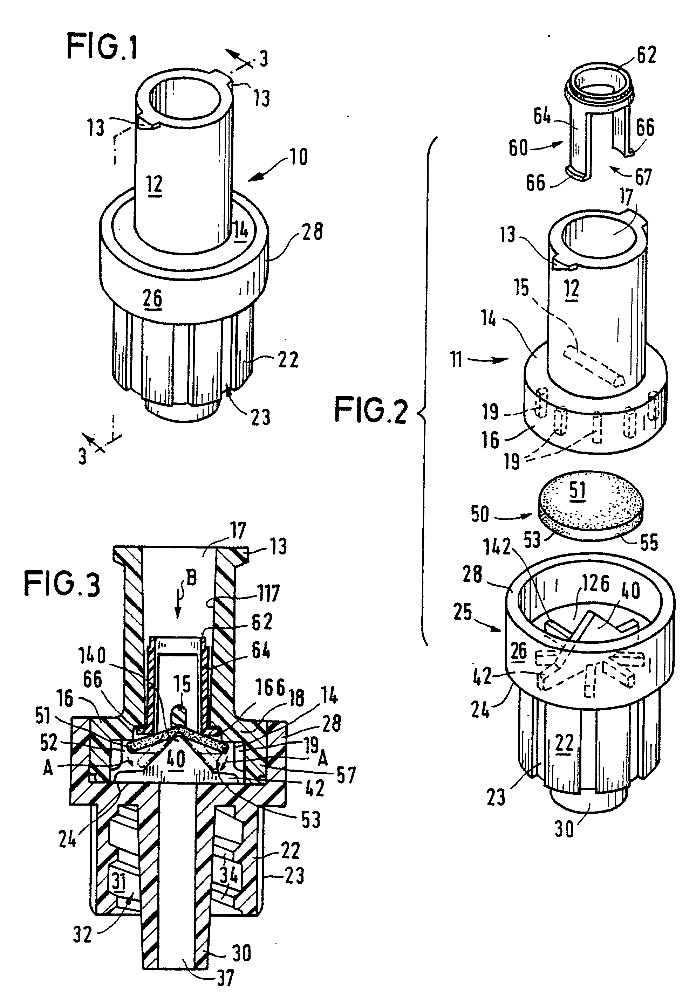

In der Kammer 52 ist eine kreisförmige Ventilscheibe 50 mit parallelen ebenen Oberflächen 51 und 53 angeordnet, die aus flexiblem gummielastischem Material hergestellt und durch Eigenelastizität rückstellfähig ist. Ferner befindet sich in der Kammer 52 ein dreieckiger Plattenkörper 40, dessen Dreieck-Basis mit nach beiden Seiten gerichteten Fortsätzen 42 mit dem Boden 24 der Kammer 52 einstückig ausgebildet ist. Von beiden Flächen des Plattenkörpers 40 gehen sternförmig weitere Stege 142 aus, die ebenfalls mit dem Boden 24 der Kammer 52 einstückig geformt sind. Die Fortsätze 42 und die Stege 142 enden mit Abstand vor der Innenfläche der Wand 57 des Gehäuseteils 11. Auf der leicht abgerundeten Spitze des Plattenkörpers 40 liegt das Zentrum der Ventilscheibe 50. Der untere ebene Rand des Querbalkens 15 drückt den Mittelbereich der Ventilscheibe 50 fest gegen die Spitze des Plattenkörpers 40. Vorzugsweise wird ein derartiger Druck ausgeübt, daß die Dreiecksspitze eine kleine Einbuchtung 140 (Fig.3) in der Ventilscheibe 50 verursacht. Diese Einbuchtung 140 hält die Ventilscheibe 50 anschließend in zentrierter Position und sichert sie gegen seitliche Bewegungen. Sollte dennoch eine Seitenbewegung auftreten oder sich bei der Montage der Rückschlagventilvorrichtung eine geringfügige Verschiebung ergeben, so verhindern die Längsrippen 19 an der Innenfläche der Wand 57 ein Anhaften oder Festklemmen des Umfangsrandes 55 der Ventilscheibe 50 an der Kammerinnenfläche. Die Spitze des Plattenkörpers 40 liegt im wesentlichen in der Ebene der Unterkante der Auskehlung 166 des Gehäuseteiles 11, so daß die Oberseite des Randbereiches der Ventilscheibe 50 in Schließstellung mit gewisser Vorspannung gegen die Unterkante der Auskehlung 166 anliegt, die als Ventilkörpersitz dient.

Bei Anschluß des Stutzens 12 an eine Infusionsvorrichtung drückt der Flüssigkeitsstrom in Richtung des Pfeiles B den Randbereich der Ventilscheibe 50 von der Unterkante der Auskehlung 166 gegen die Seiten des Dreiecks des Plattenkörpers 40 weg und ein freier Flüssigkeitsstrom in Richtung der Pfeile A durchströmt den Durchlaßkanal 117 von dem Einlaßabschnitt 17 zu dem Auslaßabschnitt 37. Um jedoch vor Anschluß einer Infusionsvorrichtung, beispielsweise zum Zuspritzen oder zum Abziehen von Flüssigkeiten, die Rückschlagventilvorrichtung öffnen zu können, ist ein Bügel 60 vorgesehen, der als Betätigungsteil für die Ventilscheibe 50 wirksam ist. Der Bügel 60, der vorzugsweise aus Kunststoff gefertigt ist, besteht aus einem kreisförmigen Ring 62, an dessen eine Seite zwei einander diametral gegenüberliegende Schenkel 64 einstückig angeformt sind. Die beiden Schenkel 64 stehen im wesentlichen senkrecht von dem Ring 62 ab, sie sind im Verhältnis zum Umfang des Ringes 64 schmal und weisen an ihrem freien Ende jeweils eine nach außen gerichtete Rippe 66 auf. Der Durchmesser des Ringes 62 entspricht dem Durchmesser des Einlaßabschnittes 17 des Durchlaßkanals 117 an seiner engsten Stelle, d.h. an der Einmündung in die Kammer 52 (Fig. 3). Die Außenfläche des Ringes 62 ist mit der Außenfläche der Schenkel 64 bündig, so daß sich bis auf die Rippen 66 eine glatte vorsprungslose Umfangsfläche des Bügels 60 ergibt. Die Rippen 66 der Schenkel 64 ragen in die Auskehlung 166 hinein und untergreifen in der in Fig. 3 gezeigten Position die Oberkante der Auskehlung 166, so daß der Bügel 60 gegen Herausziehen bzw. Herausfallen aus dem Einlaßabschnitt 17 gesichert ist. In dieser oberen Position des Bügels 16 berührt er die Oberseite der Ventilscheibe 50 nicht und diese verharrt in ihrer Schließstellung.

Zum Öffnen der Rückschlagventilvorrichtung wird der Kegel einer Spritze an den Ring 62 angesetzt und es wird durch Vorschub des Spritzenkegels der Bügel 60 in Richtung des Pfeiles B verschoben. Diese Verschiebung bewirkt, daß die Enden der Schenkel 64 gegen die Oberseite des elastisch verformbaren Randbereiches der Ventilscheibe 50 drücken und ihn von der Unterkante der Auskehlung 166 wegschieben, so daß der Durchlaß geöffnet wird. Die Position der Schenkelenden und die Ventilscheibenverformung bei maximalem Öffnungszustand ist in Fig. 3 gestrichelt angedeutet. Die Unterfläche der Ventilscheibe 50 liegt in diesem Zustand gegen die beiden Seiten des Dreiecks des Plattenkörpers 40 an. Es kann nun mit Hilfe der Spritze beliebig medikamentöse Flüssigkeit in den Durchlaßkanal 117 eingespritzt oder Körperfluid aus dem Patienten abgezogen werden. Nach Beendigung dieses Vorganges wird die Spritze zurückgezogen und die Rückstellkraft der Ventilscheibe 50 bringt ihren Randbereich in Schließstellung zurück und verschiebt den Bügel 60 in seine obere Grundstellung. Wird nun die Rückschlagventilvorrichtung an eine Infusionsvorrichtung angekoppelt, so kann der Flüssigkeitsstrom ungehindert durch den Bügel 60 die Ventilscheibe zur Öffnung des Durchlasses 117 verformen. Bei rückströmender Flüssigkeit veranlaßt diese gemeinsam mit der Rückstellfähigkeit des Ventilscheibenmaterials ein sofortiges Zurückschnellen der Ventilscheibe 50 in ihre im wesentlichen ebene Grundform mit der Folge eines unverzüglichen Verschlusses des Durchlaßkanals 117 bei Flüssigkeitsrückstrom. Der Bügel 60 hindert auch diesen Vorgang nicht, weil die verdickten Enden seiner Schenkel 64 in der Auskehlung 166 mit Abstand oberhalb der Oberseite der Ventilscheibe 50 versenkt sind.

Bei der Montage der Rückschlagventilvorrichtung wird der Bügel 60 so in den Einlaßabschnitt 17 des Gehäuseteiles 11 eingesetzt, daß seine beiden Schenkel 64 den Querbalken 15 flankieren, ihn jedoch nicht berühren. Da der Querbalken 15 zu der Ebene des Plattenkörpers 40 unter einem Winkel, vorzugsweise einem Winkel von 90°, ausgerichtet ist, liegen die beiden Schenkel 64 mit der Ebene des Plattenkörpers 40 in gemeinsamer Flucht und bei Niederdrücken des Bügels 60 dienen die beiden Seiten des Dreiecks des Plattenkörpers 40 als Anschlag für die Ventilscheibe 50, so daß sie nicht über das durch den spitzen Winkel des Dreiecks des Plattenkörpers 40 definierte Maß hinaus deformiert wird. Auf diese Weise wird die Rückstellfähigkeit der Ventilscheibe 50 über längere Zeit zuverlässig aufrechterhalten.

Die ineinandersteckenden Ansätze 16 und 26 der beiden Gehäuseteile 11 und 25 werden nach dem Einbau der Teile vorzugsweise durch Ultraschallschweißung fest und unlösbar miteinander verbunden. Die zusammengefügte und verschweißte Vorrichtung läßt sich nur durch Zerstörung des Ventilgehäuses 10 auseinandernehmen. Da die Ventilscheibe 50 zwischen der Spitze des Plattenkörpers 40 und dem ebenen unteren Rand des Querbalkens 15 eingespannt ist, bleibt sie stets zentriert und kann sich nicht in Seitenrichtung bewegen. Hierdurch wird eine sehr wirksame Funktion der Ventilscheibe 50 sowohl beim Öffnen durch Flüssigkeitsstrom oder mit Hilfe des Bügels 60 als auch bei der Rückschlagfunktion bei Flüssigkeitsrückstrom erzielt.

**Patentansprüche**

1. Rückschlagventilvorrichtung, bestehend aus einem hülsenförmigen Ventilgehäuse (10) mit einem an beiden Enden offenen Durchlaßkanal (117), der einen Einlaßabschnitt (17) und einen Auslaßabschnitt (37) aufweist, zwischen denen eine erweiterte und umfangsmäßig geschlossene Kammer (52) ausgebildet ist, in der eine durchbrechungslose Ventilscheibe (50) aus elastischem Material angeordnet ist, deren Zentrum zwischen der Spitze eines dreieckigen Plattenkörpers (40) und einem dieser gegenüberliegenden Querbalken (15) eingeklemmt ist und deren Randbereich in Schließstellung gegen eine Gehäuseschulter anliegt, **dadurch gekennzeichnet, daß** in dem Einlaßabschnitt (17) des Durchlaßkanals (117) mit kreisförmigem Querschnitt ein axial verschiebbarer Bügel (60) angeordnet ist, der aus einem kreisförmigen Ring (62) besteht, an dessen eine Seite der Kegel einer Spritze ansetzbar ist und von dessen anderer Seite zwei einander gegenüberliegende Schenkel (64) senkrecht abstehen, die die beiden Längsränder des Querbalkens (15) mit Abstand flankieren und deren freie Enden gegen den Randbereich der Ventilscheibe (50) andrückbar sind.

2. Rückschlagventilvorrichtung nach Anspruch 1, **dadurch gekennzeichnet, daß** die freien Enden der Schenkel (64) nach außen gerichtete vorspringende Rippen (66) aufweisen, die in eine Umfangsauskehlung (166) an der Kante einer Gehäuseschulter (14) zwischen der Kammer (52) und dem Einlaßabschnitt (17) des Durchlaßkanals (117) eingreifen.

3. Rückschlagventilvorrichtung nach Anspruch 1 oder 2, **dadurch gekennzeichnet, daß** das Ventilgehäuse (10) aus einem ersten und einem zweiten Gehäuseteil (11; 25) besteht, die jeweils einen zylindrischen becherförmigen Ansatz (16; 26) aufweisen; daß der Außendurchmesser des ersten zylindrischen Ansatzes (16) geringfügig kleiner ist als der Innendurchmesser des zweiten zylindrischen Ansatzes (26) und beide Ansätze (16,26) zur Bildung der geschlossenen zylindrischen Kammer (52) zusammengesteckt sind; daß der erste Gehäuseteil (11) den Einlaßabschnitt (17) des Durchlaßkanals (117) und den Übergang zu der Kammer (52) mit dem Querbalken (15) bildet, und daß auf dem Boden des becherförmigen Ansatzes (26) des zweiten Gehäuseteiles (25) die Basis des dreieckigen Plattenkörpers (40) angeordnet ist, der im wesentlichen in der Ebene der beiden Schenkel (64) des Bügels (60) ausgerichtet ist.

4. Rückschlagventilvorrichtung nach einem der Ansprüche 1 - 3, **dadurch gekennzeichnet, daß** die beiden Gehäuseteile (11; 25) aus glasklarem Kunststoff hergestellt sind und daß der Bügel (60) aus farbigem, undurchsichtigem Kunststoff besteht.

**Claims**

1. A check valve device consisting of a jacket-shaped valve housing (10) with a passage channel (117) open at both ends and having an inlet portion (17) and an outlet portion (37) between which an enlarged and circumferentially closed chamber (52) is formed in which an elastic material valve disc (50) free of openings is disposed, the centre of which is clamped between the tip of a triangular plate body (40) and a transversal bar (15) opposite thereof, and the edge portion of which abuts a shoulder of the housing, when in the closed position,

characterised in that

in said inlet portion (17) of said passage channel (117) of circular cross section, there is arranged an axially displaceable bracket (60) consisting of a circular ring (62), to the one side of which the cone of a syringe may be applied and from the other side of which two mutually opposite legs (64) protrude perpendicularly flanking the two logitudinal edges of said transversal bar (15) at a distance, their free ends being adapted to be pressed against the edge portion of said valve disc (50).

2. The check valve device of claim 1, characterised in that said free ends of said legs (64) have outward directed projecting ribs (66) that engage a circumferential recess (166) at the edge of a housing shoulder (14) between said chamber (52) and said inlet portion (17) of said passage channel (117).

3. The check valve device of claim 1 or 2, characterised in that said valve housing (10) consists of a first and a second housing part (11; 25), each having a cylindrical cup-shaped extension (16; 26); that the outer diameter of said first cylindrical extension (16) is slightly smaller than the inner diameter of said second cylindrical extension (26) and both extensions (16; 26) are assembled for forming said closed cylindrical chamber (52); that said first housing part (11) forms said inlet portion (17) of said passage channel (117) and the transition to said chamber (52) with said transversal bar (15) and that, on the bottom of said cup-shaped extension (26) of said second housing part (25), there is arranged the base of said triangular plate body (40) that is substantially oriented with the plane of said two legs (64) of said bracket (60).

4. The check valve device of one of claims 1-3, characterised in that said two housing parts (11; 25) are made of clear plastic material and that said bracket (60) is made of colored opaque plastic material.

**Revendications**

1. Dispositif de soupape formant clapet anti-retour constitué d'un corps de soupape (10) en forme de douille comportant un canal de passage (117) qui est ouvert aux deux extrémités et qui présente un tronçon d'entrée (17) ainsi qu'un tronçon de sortie (37), entre lesquels est formée une chambre (52) élargie et close sur la périphérie, dans laquelle est logée un disque formant clapet (50) sans évidements de passage et réalisé en un matériau élastique, dont le centre est enserré entre la pointe d'un corps en forme de plaque triangulaire (40) et une traverse (15) qui lui est opposée, et dont la zone de bordure est appliquée contre un épaulement du corps de soupape, en position de fermeture, caractérisé en ce que dans le tronçon d'entrée (17) du canal de passage (117) de section circulaire, est disposé un étrier (60) susceptible de coulisser en direction axiale et qui est constitué par un anneau de forme circulaire (62) sur l'un des côtés duquel il est possible de mettre en place le cône d'une seringue, et sur l'autre côté duquel font saillie perpendiculairement deux branches opposées (64) qui viennent flanquer à distance les deux bords longitudinaux de la traverse (15) et dont les extrémités libres peuvent être pressées contre la zone de bordure du disque formant clapet (50).

2. Dispositif de soupape formant clapet antiretour selon la revendication 1, caractérisé en ce que les extrémités libres des branches (64) comportent des nervures (66) en saillie, dirigées vers l'extérieur, et qui s'engagent dans une gorge périphérique (166) sur le bord d'un épaulement (14) du corps de soupape, entre la chambre (52) et le tronçon d'entrée (17) du canal de passage (117).

3. Dispositif de soupape formant clapet anti-retour selon la revendication 1 ou 2, caractérisé en ce que le corps de soupape (10) se compose d'une première et d'une seconde partie de corps de soupape (11;25) qui comportent chacune une embase cylindrique (16;26) en forme de cuvette, en ce que le diamètre extérieur de la première embase cylindrique (16) est très légèrement inférieur au diamètre intérieur de la seconde embase cylindrique (26), les deux embases (16,26) étant emmanchées l'une dans l'autre pour former la chambre cylindrique fermée (52), en ce que la première partie de corps de soupape (11) forme le tronçon d'entrée (17) du canal de passage (117) et la transition vers la chambre (52) avec la traverse (15), et en ce que sur le fond de l'embase (26) en forme de cuvette de la seconde partie de corps de soupape (25), est disposée la base du corps en forme de plaque triangulaire (40) qui est orienté de manière à se trouver sensiblement dans le plan des deux branches (64) de l'étrier (60).

4. Dispositif de soupape formant clapet antiretour selon l'une des revendications 1 à 3, caractérisé en ce que les deux parties de corps de soupape (11;25) sont réalisées en une matière synthétique transparente, et en ce que l'étrier (60) est réalisé en une matière synthétique colorée, non transparente.

FIG.1

FIG.2

FIG.3